# EUROPÄISCHE PATENTSCHRIFT

(11) **EP 1 162 955 B1**
(45) Veröffentlichungstag und Bekanntmachung des Hinweises auf die Patenterteilung: **15.06.2005**
(21) Anmeldenummer: 00922528.5
(22) Anmeldetag: 17.03.2000
(51) Int. Cl.: A61K 9/51, A61K 47/48, C12N 15/87, C12N 15/88, A61K 48/00

(54) **NANOSKALIGE TEILCHEN, KOMPLEXE MIT POLYNUKLEOTIDEN UND DEREN VERWENDUNG**
NANOPARTICLES, COMPLEXES WITH POLYNUCLEOTIDES AND THEIR USE
NANOPARTICULES, LEURS COMPLEXES AVEC DES POLYNUCLEOTIDES ET LEUR UTILISATION

(30) Priorität: 19.03.1999 DE 19912502
(43) Veröffentlichungstag der Anmeldung: 19.12.2001
(73) Patentinhaber: INSTITUT FÜR NEUE MATERIALIEN GEM. GMBH, 66123 Saarbrücken (DE); Across Barriers Gesellschaft für neue Technologien zur Absorption und Applikation von Arzneistoffen MBH, 66123 Saarbrücken (DE)
(72) Erfinder: SCHIESTEL, Thomas, D-70565 Stuttgart (DE); SCHIRRA, Hermann, D-66113 Saarbrücken (DE); SCHMIDT, Helmut, D-66130 Saarbrücken-Güdingen (DE); LEHR, Claus-Michael, D-66125 Saarbrücken-Dudweiler (DE); HALTNER, Eleonore, D-66121 Saarbrücken (DE); KNEUER, Carsten, D-15299 Müllrose (DE); SAMETI, Mohammad, D-66121 Saarbrücken (DE)
(74) Vertreter: Barz, Peter
(86) Internationale Anmeldenummer: PCT/EP2000/002409
(87) Internationale Veröffentlichungsnummer: WO 2000/056288

(56) Entgegenhaltungen:
- WO-A-97/38058
- SCHIESTEL T, ET AL.: "Development of a flexible cell targeting system based on silica nanoparticles" MATERIALS RESEARCH SOCIETY SYMPOSIUM PROCEEDINGS, Bd. 530, 1998, Seiten 65-71, XP000911914 ISSN: 0272-9172
- CHRISEY LA, ET AL.: "Selective attachment of synthetic DNA to self-assembled- monolayer functionalized surfaces" MATERIALS RESEARCH SOCIETY SYMPOSIUM PROCEEDINGS, Bd. 330, 1994, Seiten 179-184, XP000614716 ISSN: 0272-9172
- SCHMIDT HK, ET AL.: "Organic-inorganic hybrid materials processing and applications" MATERIALS RESEARCH SOCIETY SYMPOSIUM PROCEEDINGS, Bd. 576, 5. April 1999 (1999-04-05), Seiten 395-407, XP000897942 ISSN: 0272-9172
- KNEUER C, ET AL.: "Silica nanoparticles modified with aminosilanes as carriers for plasmid DNA" INTERNATIONAL JOURNAL OF PHARMACEUTICS (SPECIAL ISSUE ON "COLLOIDAL DRUG CARRIERS", JUNE 3-5TH 1999), Bd. 196, 10. März 2000 (2000-03-10), Seiten 257-261, XP000900562 ISSN: 0378-5173

## Beschreibung

Die Erfindung betrifft Nanopartikel-Polynukleotid-Komplexe von nanoskaligen Teilchen mit einem Kern aus anorganischen Primärpartikeln, deren Oberfläche durch Reaktion mit einem funktionelle Gruppen enthaltenden Organosilan oder Polyorganosiloxan vollständig oder teilweise mit einer Hülle belegt ist. Die Erfindung betrifft Komplexe dieser nanoskaligen Teilchen mit Polynukleotiden, typischerweise DNA, sowie derartige Komplexe enthaltende pharmazeutische Zusammensetzungen und die Verwendung der nanoskaligen Teilchen bzw. deren Polynukleotid-Komplexe zur Transfektion von Targetzellen bzw. in der Gentherapie.

Materialoberflächen reagieren bei Berührung mit Flüssigkeiten häufig durch einen Ladungsaustausch. Dies ist besonders dann der Fall, wenn die Flüssigkeiten prototrop und die Oberflächen polar sind, wie dies häufig bei anorganischen Materialien wie z. B. Oxiden der Fall ist. Die Aufladung der Oberfläche wird als Zeta-Potential bezeichnet. Bei kleinen Partikeln, z. B. Kolloiden, kann die Aufladung der Oberfläche dazu führen, daß sich solche Partikel einander nicht mehr annähern und damit stabilisiert werden. Diese Stabilisierung hängt von der Größe des Zeta-Potentials (Ladung/Oberflächeneinheit) ab und kann sowohl positiv als auch negativ sein. In prototropen Flüssigkeiten, bei denen ein pH-Wert definiert ist, läßt sich die Aufladung durch den pH-Wert einstellen (Zeta-Potentialkurve). Weiterhin hängt die Aufladung von der chemischen Natur der Oberfläche als Säure oder Base ab, die als Funktion des pH-Wertes mehr oder weniger dissoziiert sind und damit das Potential bestimmen. Bei vorgegebenen Materialien (z. B. SiO₂, ZrO₂, TiO₂) sind das Zeta-Potential und die Zeta-Potentialkurve eine feste Funktion der Chemie der Partikeloberfläche und können nur über den pH-Wert in ihrer Größe verschoben werden. Hinzu kommt, daß die Gesamt-Ionenkonzentration einer Lösung oder der flüssigen Phase noch eine Rolle spielt. Diese kolloidchemischen Grundlagen sind allgemein bekannt, und man weiß, daß über die geschilderten Prinzipien die Oberflächenladung und das Zeta-Potential einstellen werden können. Da jedoch der pH-Wert oft nicht frei wählbar ist, wäre eine Methode wünschenswert, die unabhängig vom pH-Wert die Einstellung der Ladungsdichte der Oberfläche gestattet.

In der rekombinanten Gentechnologie und der Gentherapie werden Targetzellen transfiziert, indem man die gewünschte DNA in die Zellen einschleust. Hierzu muß die DNA an einen Träger gebunden werden, der die DNA durch Ladungkompensation und Verringerung iherer räumlichen Ausdehnung (DNA-Kondensation) in eine transportfähige Form bringt. Durch eine Funktionalisierung des Trägersystems mit geeigneten Liganden kann eine effiziente und spezifische Bindung an Targetzellen mit einem anschließenden intrazellulären Transport in den Zellkern erreicht werden.

Schiestel et al., Materials Research Society Symposium Proceedings, Bd. 530, 1998, Seiten 65 bis 71, beschreiben nanoskalige Teilchen mit einem Durchmesser von 10 bis 80 nm mit einem Kern aus SiO₂, deren Oberfläche mit einem Aminosilan modifiziert sein kann, wobei die Teilchen ein Zeta-Potential zwischen +30 und +35 mV unter physiologischen Bedingungen aufweisen. Weiter wird beschrieben, dass Avidin, ein. Glycoprotein, an negativ geladene Nanopartikel gebunden werden kann. Ferner wird die Anbindung von biotinyliertem WGA an Partikel beschrieben, wobei entweder über einen Avidin/Biotin-Komplex oder direkt an negativ geladene Partikel gebunden wird.

WO 97/38058 beschreibt Suspensionen von stabil beschichteten nanoskaligen Eisenoxidteilchen. Die Teilchen können einen Durchmesser von 40 bis 100 nm und einen Überzug auf Aminosilan-Basis aufweisen. Die mit Aminosilanen oberflächenmodifizierten Teilchen weisen einen isoelektrischen Punkt bei pH 8,8 bis 9,6 auf. Weiter wird die Kopplung von Verbindungen, wie Kronenethem, Komplexbildnern. Biomolekülen, an funktionelle Gruppen der Teilchen erwähnt.

Aufgabe der Erfindung war es, nanoskalige Teilchen bereitzustellen, deren Oberflächenladungen und Oberflächenladungsdichten unabhängig vom pH und unabhängig von der Oberfläche eingestellt werden können. Dabei sollten Oberflächenladungen im neutralen pH-Bereich (7,0-7,5) erzielt werden, die für physiologische Anwendungen erforderlich sind, so daß eine Verwendung als DNA-Trägersystem zur Transfektion von Targetzellen ins Auge gefaßt werden konnte.

Eine weitere Aufgabe bestand darin, Nanoteilchen-Polynukleotid-Komplexe bereitzustellen, die eine Stabilisierung von Polynukleotiden ermöglichen und in der rekombinanten Gentechnologie oder Gentherapie zur Transfektion von Targetzellen geeignet sind.

Gegenstand der Erfindung sind Nanopartikel-Polynukleotid-Komplexe, bei denen, nanoskalige Teilchen mit einem Kern aus anorganischen Primärpartikeln, deren Oberfläche durch Reaktion mit einem funktionelle Gruppen enthaltenden Organosilan oder Polyorganosiloxan vollständig oder teilweise mit einer Hülle belegt ist, wobei die Teilchen im neutralen pH-Bereich von 7,0 bis 7,5 ein Zeta-Potential von 0 bis + 100 mV, vorzugsweise 0 bis 60 mV, aufweisen, an Polynukleotide gebunden sind. Gegenstand der Erfindung sind auch Suspensionen, die derartige Partikel in einem wäßrigen, wäßrig-organischen oder organischen Lösungsmittelsystem suspendiert enthalten.

Gegenstand der Erfindung sind femer Nanopartikel-Polynukleotid-Komplexe, bei denen ein Polynukleotid (DNA, RNA, Oligonukleotide, Analoga) an ein derartiges nanoskaliges Teilchen gebunden ist.

Die Erfindung betrifft ferner pharmazeutische Zusammensetzungen, die die genannten Nanopartikel-Polynukleotid-Komplexe neben pharmazeutisch verträglichen Träger- und/oder Hilfsstoffen enthalten, sowie die Verwendung der nanoskaligen Teilchen bzw. Nanopartikel-Polynukleotid-Komplexe zur stabilen Lagerung von Polynukleotiden und für den spezifischen oder unspezifischen Transfer genetischer Information in vorzugsweise eukaryontische Targetzellen (Transfektion). In diesem Rahmen eignen sich die nanoskaligen Teilchen und Nanopartikel-Polynukleotid-Komplexe z.B. zur lokalen, oralen oder systemischen Behandlung von Krankheiten und zum Einsatz in der Gentherapie.

Beispielsweise kann ein Gen für eine therapeutisch aktive Substanz, wie das Gen für das CFTR-Protein, den LDL-Rezeptor oder die Thymidinkinase, mit Hilfe der genannten Nanoteilchen in eine geschützte, biokompatible und aufnahmefähige Form überführt werden. Die Nanopartikel-Polynukleotid-Komplexe der Erfindung gestatten z.B. die gentherapeutische Behandlung entzündlicher Schleimhauterkrankungen, wenn man Antisense-Oligonukleotide zur Unterdrückung der Proliferation von Entzündungsmediatoren, z.B. Interleukinen, an die erfindungsgemäßen Nanoteilchen zum Zwecke der Stabilisierung und Vermittlung der zellulären Aufnahme bindet.

Die erfindungsgemäßen Nanopartikel-Polynukleotid-Komplexe lassen sich reproduzierbar herstellen und weisen eine geringe Polydispersität auf. Die an die Nanoteilchen gebundenen Polynukleotide sind sowohl vor physikalischer Beanspruchung als auch physiologischem (z.B. enzymatischem) Abbau geschützt. Es handelt sich somit um "sichere" Systeme, die nicht die Risiken viraler Trägersysteme in sich bergen und im Vergleich zu organischen Polymerträgern eine unerwartet niedrige Cytotoxizität zeigen.

Die nanoskaligen Teilchen weisen vorzugsweise eine Teilchengröße von 2 bis 150 nm, insbesondere 2 bis 100 nm, auf.

Die anorganischen Primärpartikel der nanoskaligen Teilchen sind vorzugsweise ausgewählt aus Metalloxiden wie Magnetit (Fe₃O₄), Maghemit (γ-Fe₂O₃), Mischoxiden der Formel Me(II)Fe₂O₄, in der Me(II) Zn, Cu, Co, Ni oder Mn repräsentiert, Al₂O₃ (z.B. Boehmit), TiO₂, ZrO₂; Nichtmetalloxiden wie SiO_{2.} Calciumcarbonat, Calciumphosphaten wie Hydroxylapatit oder Tricalciumphosphat, oder Borverbindungen wie Boroxid oder borhaltigen Metalllegierungen (z.B. Fe-B-Legierungen). Weitere Beispiele für geeignete anorganische Primärpartikel sind z.B. in den DE-A-195 12 427.8, DE-A-195 15 820.2 und DE-A-196 13 645.8 beschrieben. Dazu zählen unter anderem Oxide, Oxidhydrate, Sulfide, Selenide, Telluride, Halogenide, Nitride, Carbide, Carbonitride, Boride, Silicide, Arsenide, Antimonide, Phosphide, Carbonate, Carboxylate, Phosphate, Sulfate, Silikate, Titanate, Zirkonate, Aluminate, Stannate, Plumbate und Mischoxide von Si, Al, B, Zn, Cd, Ti, Zr, Ce, Sn, In, La, Fe, Pb, Cu, Ta, Nb, V, Mo, W, Alkali- oder Erdalkalimetallen.

Die anorganischen Primärpartikel haben vorzugsweise eine Teilchengröße von 1 bis 150 nm, insbesondere 5 bis 100 nm. Die Hülle aus Organosilan oder Polyorganosiloxan hat je nach Belegung eine Schichtdicke ausgehend von z.B. 0,5 bis 1,5 nm (Monolayer) bis zu vorzugsweise 50 nm, insbesondere bis zu 30 nm.

Die anorganischen Primärpartikel werden z.B. auf die in WO 97/38058 beschriebene Weise ganz oder teilweise mit einer Hülle aus einem Organosilan oder Polyorganosiloxan mit funktionellen Gruppen versehen. Zu diesem Zweck geeignete Ausgangssilane sind z.B. funktionelle Organosilane mit 1, 2 oder 3 hydrolysierbaren Gruppen, z. B. Mono-, Di- oder Trialkoxysilane, deren Alkoxygruppen vorzugsweise identisch sind und 1 bis 4 Kohlenstoffatome enthalten, entsprechende Chlorsilane und Silazane. Die anderen an das Silicium gebundenen Gruppen sind Kohlenwasserstoffgruppen, z.B. Alkylgruppen, die vorzugsweise über 1 bis 20, besonders bevorzugt 1 bis 10 und insbesondere 1 bis 4 Kohlenstoffatome verfügen. Mindestens eine dieser Kohlenwasserstoffgruppen ist hierbei eine aliphatische oder cycloaliphatische Gruppe, die durch eine oder mehrere Gruppen -O- und/oder -NR- (R = H oder C₁₋₄-Alkyl) unterbrochen sein kann und auch eine oder mehrere Kohlenstoff-Kohlenstoff-Doppel- oder -Dreifachbindungen umfassen kann. Diese Kohlenwasserstoffgruppe ist über eine Si-C-Bindung an das Siliciumatom geknüpft. Weiter muß diese Kohlenwasserstoffgruppe über mindestens eine Amino-, Carboxyl-, Epoxy-, Mercapto-, Cyano-, lsocyanato-, Hydroxy-, Vinyl- und/oder (Meth)acrylgruppe verfügen, wobei die Gruppen auch Derivate einschließen (z.B. Ester, Anhydrid und Säurehalogenid im Fall von Carboxyl und Mono- und Dialkylamino sowie Trialkylammonium im Fall von Amino). Selbstverständlich können auch zwei oder mehr unterschiedliche funktionelle Gruppen an eine derartige Kohlenwasserstoffgruppe gebunden sein. Erfindungsgemäß bevorzugt ist der Einsatz von Organosilanen mit mindestens einer Aminogruppe (im folgenden als Aminosilane bezeichnet). Konkrete Beispiele für derartige Aminosilane sind 3-Aminopropyltriethoxysilan, N-(2-Aminoethyl-3-aminopropyltrimethoxysilan, Trimethoxysilylpropyldiethylentriamin und N-(6-Aminohexyl)-3-aminopropyltrimethoxysilan.

Bei der Belegung der anorganischen Primärpartikel mit Organosilanen reagieren die hydrolysierbaren Gruppen und funktionellen Gruppen des Organosilans mit reaktiven Oberflächengruppen (z.B. Hydroxylgruppen) der Primärpartikel und bewirken eine feste Verankerung der Hülle. Im Falle der Belegung mit Polyorganosiloxanen erfolgt eine hydrolytische Polykondensation der Organosilane in Gegenwart der anorganischen Primärpartikel, wobei diese vollständig oder teilweise mit einer Polyorganosiloxan-Hülle beschichtet werden.

Die verwendeten Organosilane können gegebenenfalls vor der Belegung der Primärpartikel mit Derivatisierungsmitteln umgesetzt werden, um die vorhandenen funktionellen Gruppen zu derivatisieren. Bei diesen Derivatisierungsmitteln handelt es sich z.B. um polyfunktionelle Amine, beispielsweise Polyamine, die gegebenenfalls ganz oder teilweise alkyliert sind, wie Ethylendiamin und dessen Oligomere und Polymere, Ethylenimin und dessen Oligomere und Polymere, basische Aminosäuren oder Proteine, insbesondere Lysin, Arginin und deren Oligomere und Polymere, 2,4,6-Triaminopyrimidin oder Melamin.

In ähnlicher Weise können im Falle der Belegung der Primärpartikel mit Polyorganosiloxanen die erhaltenen nanoskaligen Teilchen mit Kern-Hüllen (Core-Shell)-Struktur gegebenenfalls mit Modifizierungsmittein umgesetzt werden, um die funktionellen oder derivatisierten Gruppen des Polyorganosiloxans zu modifizieren. Als derartige Modifizierungsmittel eignen sich die vorstehend genannten Derivatisierungsmittel.

Vorzugsweise werden die funktionellen Gruppen, Derivatisierungsmittel und Modifizierungsmittel (summarisch: "Oberflächenmodifikatoren") so ausgewählt, daß die nanoskaligen Teilchen eine positive Oberflächenladung aufweisen. Die oberflächenchemischen Eigenschaften der nanoskaligen Teilchen lassen sich durch die Art und Menge der Oberflächenmodifikatoren in einem weiten Bereich steuern. Beispielsweise läßt sich der isoelektrische Punkt (IEP) der Teilchen durch Variation des Verhältnisses von Partikel zu Oberflächenmodifikator im Bereich zwischen dem IEP der unbeschichteten Partikel und dem IEP der vollständig beschichteten Partikel einstellen. Der IEP-Wert der vollständig beschichteten Partikel wird durch den pK-Wert der funktionellen Gruppen der Beschichtung festgelegt. Die Ladungsdichte und damit die Bindungskapazität für entgegengesetzt geladene Stoffe läßt sich in einem breiten pH-Bereich steuern, indem die zur Belegung der Primärpartikel verwendeten Organosilane oder Polyorganosiloxane durch Wahl entsprechender Polyamine mit einer definierten Anzahl funktioneller Gruppen derivatisiert werden.

Im Falle von Nanopartikel-Polynukleotid-Komplexen erschwert eine Reihe spezifischer Eigenschaften der Polynukleotide den Umgang mit diesen Substanzen. Dies sind insbesondere auf die stark negative Ladung, die physikalische Instabilität, die enzymatische Instabilität und die räumliche Ausdehnung. Polynukleotide tragen unter physiologischen Bedingungen eine dem Polymerisationsgrad entsprechende Anzahl negativer Ladungen (Phosphatgruppen). Durch elektrostatische Abstoßung bewirken diese Ladungen eine Raumausdehnung der Polynukleotide, die für Expressionsplasmide im Mikrometerbereich liegt. Bei einer mindestens 50 % igen Kompensation der negativen Ladungen können diese eine andere, dichtere Struktur mit geringerer räumlicher Ausdehnung annehmen. Im Falle der DNA kommt es dabei zum Kollabieren der räumlichen Struktur, ein Vorgang, der nachfolgend als "DNA-Kondensation" bezeichnet wird. Hierbei handelt es sich um einen hydrophoben Kollaps des interpolyelektrolytischen Komplexes zwischen der DNA und dem polykationischen Nanopartikel, wenn eine ausreichende Anzahl von negativen Ladungen im DNA-Gerüst kompensiert worden ist.

Die DNA-Kondensation wird erfindungsgemäß mit positiv geladenen Nanopartikeln erreicht, wodurch die Polynukleotide physikalisch stabilisiert und gegen den Angriff von Enzymen (Nukleasen) geschützt sind. Durch gezielte Oberflächenmodifikation der Nanopartikel läßt sich deren Zeta-Potential und damit die DNA-Bindungsstärke je nach Art und Oberflächenkonzentration (Oberflächendichte) des eingesetzten Aminosilans in einem weiten Bereich einstellen, und der isoelektrische Punkt der so hergestellten Nanopartikel kann entsprechend flexibel variiert werden. Ferner kann die Partikelgröße so optimiert werden, daß sich das Polynukleotid (z.B. DNA) ohne Spannung um die Partikel winden läßt.

Die Oberflächenladungsdichte ist wie oben beschrieben in einem weiten pH-Bereich einstellbar und läßt sich für unterschiedliche Polynukleotide optimieren. Die Steuerung der Abstände der Ladungsträger über sogenannte Spacer (variable (Cyclo)alkylketten zwischen der funktionellen Gruppe und dem Siliciumatom), führt zu einer optimalen Wechselwirkung des Polynukleotids mit den anorganischen Nanopartikein.

Durch das Etablieren einer ausreichend großen Anzahl positiver Ladungen in den richtigen Abständen können somit Polynukleotide wie DNA durch elektrostatische Wechselwirkung auf den Nanopartikeln fixiert werden (DNA-Kondensation).

Auch die Basizität läßt sich in einem weiten Bereich einstellen, was die Einstellung von Puffereigenschaften erlaubt. Diese haben unter Umständen einen starken Einfluß auf das intrazelluläre Verhalten.

Weiterhin können an die funktionellen, derivatisierten oder modifizierten Gruppen andere Moleküle angekoppelt werden, welche z.B. eine Steuerung der biologischen Transportwege ermöglichen, z.B. Lektine, Transferrin, Folsäure, Vitamin B12 und Transportproteine. Auch kleine Moleküle, wie DNase-Inhibitoren, die auf spezifische Signale hin freigesetzt werden, können angekoppelt werden.

Desweiteren kann an die Teilchenoberfläche eine Hülle aus Oberflächenmodiftkatoren (z.B. PEG) angekoppelt werden, mit der sich die Biozirkulation positiv beeinflussen läßt. Die Nanoteilchen können auch vollständig von einem liposomalen System umschlossen sein.

Bei den erfindungsgemäßen Nanopartikel-Polynukleotid-Komplexen handelt es sich um "sichere" Transportsysteme, die nicht die Risiken viraler Transportsysteme (Gefahr der Mutagenität, Bildung replikationskompetenter Viren, unzuverlässige Sicherheitstests) in sich bergen und im Vergleich zu organischen Polymerträgern eine unerwartet niedrige Cytotoxizität zeigen.

Das Gewichtsverhältnis Nanopartikel/Polynukleotid in den Komplexen kann z.B. 0,5:1 bis 500:1, vorzugsweise 1:1 bis 300:1 und insbesondere 30:1 bis 200:1, betragen.

Die folgenden Beispiele erläutern die Erfindung.

### Beispiel 1

### 1. Partikelsynthese:

Zu einer Lösung von 8 g Tetraethoxysilan (TEOS) in 250 ml Ethanol werden 13 g NH₃ conc. gegeben. Es wird 16 h bei Raumtemperatur umgesetzt und anschließend durch Dialyse gereinigt. Es resultieren Suspensionen, in denen Partikel mit einem mittleren Durchmesser von 80 nm und einem Zeta-Potential von -50 mV bei pH 7.4 vorliegen.

Durch Variation der Reaktionsparameter lassen sich die Partikeleigenschaften in einem weiten Bereich variieren.

### 2. Aminosilan-Modifizierung:

20 g einer kommerziell erhältlichen, 30 gew.-% Suspension von Silica-Partikeln in Isopropanol (IPAST, NISSAN Chemical Industries), 20 ml Wasser, 12 ml Eisessig und 6 g N-(6-Aminohexyl)-3-aminopropyltrimethoxysilan werden 16 h bei 80°C gerührt. Danach werden 25 ml Ethylenglycol zugesetzt und Wasser im Vakuum entfernt. Anschließend wird die verbleibende Suspension durch Dialyse gereinigt. Die resultierenden oberflächenmodifizierten Partikel Si26H besitzen einen hydrodynamischen Durchmesser von 26 nm und ein Zeta-Potential von + 31 mV bei pH 7.4.

Diese Reaktion läßt sich allgemein zur Modifizierung von anorganischen Nanopartikeln mit Aminosilanen einsetzen.

### 3. Herstellung von Nanopartikel-DNA-Komplexen

Zur Herstellung der Nanopartikel-DNA-Komplexe genügt es in der Regel, beide Komponenten zu mischen. Die in 2) hergestellten Partikel werden in PBS aufgenommen und als 30facher Gewichtsüberschuß mit Plasmid-DNA (pCMVβ) in PBS gemischt. Es resultieren DNA-Nanopartikel-Komplexe mit einem hydrodynamischen Durchmesser von 180 nm und einem Zeta-Potential von -50 mV bei pH 7,4. Diese Nanopartikel-DNA-Komplexe können direkt zur Transfektion verwendet werden.

### 4. Durchführung der Transfektion:

Die Transfektion wurde in vitro mit Cos-1-Zellen untersucht. Diese wurden hierzu zuerst mit DMEM (Dulbecco's Modified Eagle's Medium) und FCS (fetal calf serum) für 48 h bei 37°C und 5% CO₂ in Luft inkubiert. Die Zellen wurden dann mit den Nanopartikel-DNA-Komplexen versetzt und die Transfektion wurde über die Expression der β-Galactosidase bestimmt. Hierzu wurden die Zellen nach der Transfektion mit Triton-X lysiert und die Fluoreszenz bei 460 nm bestimmt. Die DNA konnte durch Verwendung der anorganischen Carriersysteme erfolgreich transfiziert werden.

### 5. Ankopplung von Lektinen:

1 ml einer 2 % Partikelsuspension der nach 1) hergestellten Partikel in PBS wird mit 100 µl einer Avidin-Lösung der Konzentration 60 µg/ml 1 h bei 37°C inkubiert. Durch Zentrifugation wird nicht umgesetztes Avidin abgetrennt. Anschließend wird 1 ml einer 2 % Avidin-Partikelsuspension in PBS mit 75 µl einer biotinylierten WGA-Lösung (Weizenlektin) der Konzentration 100 µg/ml 1 h bei 37°C inkubiert. Es resultieren oberflächenmodifizierte Partikel mit einem hydrodynamischen Durchmesser von 600 nm mit einem Gehalt von 2.8 µg WGA pro mg Partikel. Durch diese Oberflächenmodifzierung wird die Bindung an L-132 Zellen um den Faktor 4 verbessert, die Endocytose sogar um den Faktor 10.

Diese Reaktion läßt sich ganz allgemein zur Ankopplung biotinylierter Biomoleküle an negativ geladene Nanopartikel oder Nanopartikel-DNA-Komplexe verwenden.

### 6. Zeta-Potential-Messungen:

Die Zeta-Potentiale wurden mit dem Zetasizer 4 der Fa. Malvern durch eine Untersuchung der elektrophoretischen Mobilität bestimmt. Das Gerät wurde vor der Messung mit einem Latexstandard (- 55 mV) kalibriert.

### Beispiel 2

Die Verfahrensschritte von Beispiel 1 werden wiederholt, jedoch verwendet man anstelle der oberflächenmodifizierten Nanopartikel Si26H die in der folgenden Tabelle genannten Partikel Si10E, Si22E und Si100E. Es werden analoge Ergebnisse erzielt.

**Tabelle**

| Partikel | mittl. Größe ( nm) | IEP (pH) | ζ bei pH 7,4 (mV) | Spacer des Aminosilans |
|---|---|---|---|---|
| Si10E | 10 | 8,1 | + 17 | Ethyl |
| Si22E | 22 | 8,6 | + 26 | Ethyl |
| Si100E | 100 | 7,7 | + 7 | Ethyl |
| Si26H | 26 | 8,6 | + 31 | Hexyl |

## Patentansprüche

1. Nanopartikel-Polynukleotid-Komplexe, bei denen nanoskalige Teilchen mit einem Kern aus anorganischen Primärpartikeln, deren Oberfläche durch Reaktion mit einem funktionelle Gruppen enthaltenden Organosilan oder Polyorganosiloxan vollständig oder teilweise mit einer Hülle belegt ist, wobei die Teilchen im neutralen pH-Bereich von 7,0 bis 7,5 ein Zeta-Potential von 0 bis + 100 mV aufweisen, an Polynukleotide gebunden sind.

2. Nanopartikel-Polynukleotid-Komplexe nach Anspruch 1, **dadurch gekennzeichnet, dass** die anorganischen Primärpartikel ausgewählt sind aus Metalloxiden wie Magnetit oder Maghemit, Nichtmetalloxiden wie SiO₂, Calciumphosphaten oder Borverbindungen.

3. Nanopartikel-Polynukleotid-Komplexe nach Anspruch 1 oder 2, **dadurch gekennzeichnet, dass** die funktionellen Gruppen des Organosilans oder Polyorganosiloxans Aminogruppen sind.

4. Nanopartikel-Polynukleotid-Komplexe nach einem der Ansprüche 1 bis 3, **dadurch gekennzeichnet, dass** die verwendeten Organosilane mit Derivatisierungsmitteln derivatisiert worden sind.

5. Nanopartikel-Polynukleotid-Komplexe nach einem der Ansprüche 1 bis 4, **dadurch gekennzeichnet, dass** die funktionellen oder derivatisierten Gruppen des Organosilans oder Polyorganosiloxans mit Modifizierungsmitteln umgesetzt worden sind.

6. Nanopartikel-Polynukleotid-Komplexe nach einem der Ansprüche 1 bis 5, **dadurch gekennzeichnet, dass** die funktionellen Gruppen, Derivatisierungsmittel und Modifizierungsmittel so ausgewählt worden sind, dass die Teilchen eine positive Oberflächenladung aufweisen.

7. Nanopartikel-Polynukleotid-Komplexe nach einem der Ansprüche 1 bis 6, **dadurch gekennzeichnet, dass** die Oberflächenladungsdichte und Teilchengröße der nanoskaligen Teilchen so eingestellt sind, dass in Anwesenheit von Polynukleotiden die Struktur der Polynukleotide kollabiert und die räumliche Ausdehnung des Polynukleotids verringert wird.

8. Nanopartikel-Polynukleotid-Komplexe nach einem der Ansprüche 1 bis 7, **dadurch gekennzeichnet, dass** über die funktionellen, derivatisierten oder modifizierten Gruppen des Organosilans oder Polyorganosiloxans Moleküle angekoppelt sind, welche eine Steuerung der biologischen Transportwege ermöglichen.

9. Nanopartikel-Polynukleotid-Komplexe nach einem der Ansprüche 1 bis 8, **dadurch gekennzeichnet, dass** auf die Komplexoberfläche Oberflächenmodifikatoren aufgebracht sind, welche die Biozirkulation beeinflussen.

10. Nanopartikel-Polynukleotid-Komplexe nach einem der Ansprüche 1 bis 9, **dadurch gekennzeichnet, dass** sie von einem liposomalen System umschlossen sind.

11. Nanopartikel-Polynukleotid-Komplexe nach einem der Ansprüche 1 bis 10 für den spezifischen oder unspezifischen Transfer genetischer Information in Targetzellen.

12. Nanopartikel-Polynukleotid-Komplexe nach einem der Ansprüche 1 bis 11 zur lokalen, oralen oder systemischen Behandlung von Krankheiten.

13. Nanopartikel-Polynukleotid-Komplexe nach einem der Ansprüche 1 bis 12 zur Transfektion von Targetzellen bzw. zur Gentherapie.

14. Pharmazeutische Zusammensetzung, enthaltend mindestens einen Nanopartikel-Polynukleotid-Komplex nach einem der Ansprüche 1 bis 13 und pharmazeutisch verträgliche Excipienten und/oder Hilfsstoffe.

15. Verwendung der Nanopartikel-Polynukleotid-Komplexe nach einem der Ansprüche 1 bis 10 zur stabilen Lagerung von Polynukleotiden.

16. Verwendung der Nanopartikel-Polynukleotid-Komplexe nach einem der Ansprüche 1 bis 10 für den spezifischen oder unspezifischen Transfer genetischer Information in Targetzellen oder zur Transfektion von Targetzellen, wobei therapeutische Behandlungen ausgenommen sind.

## Claims

1. Nanoparticle-polynucleotide complexes in which nanoscale particles having a core of inorganic primary particles, whose surface is fully or partially provided with a shell by reaction with an organosilane or polyorganosiloxane containing functional groups, the particles having a zeta potential of from 0 to +100 mV in the neutral pH range of from 7.0 to 7.5, are bound to polynucleotides.

2. Nanoparticle-polynucleotide complexes according to Claim 1, **characterized in that** the inorganic primary particles are selected from metal oxides such as magnetite or maghaemite, nonmetal oxides such as SiO₂, calcium phosphates or boron compounds.

3. Nanoparticle-polynucleotide complexes according to Claim 1 or 2, **characterized in that** the functional groups of the organosilane or polyorganosiloxane are amino groups.

4. Nanoparticle-polynucleotide complexes according to one of Claims 1 to 3, **characterized in that** the organosilanes employed have been derivatized using derivatizers.

5. Nanoparticle-polynucleotide complexes according to one of Claims 1 to 4, **characterized in that** the functional or derivatized groups of the organosilane or polyorganosiloxane have been reacted with modifiers.

6. Nanoparticle-polynucleotide complexes according to one of Claims 1 to 5, **characterized in that** the functional groups, derivatizers and modifiers have been selected so that the particles have a positive surface charge.

7. Nanoparticle-polynucleotide complexes according to one of Claims 1 to 6, **characterized in that** the surface charge density and particle size of the nanoscale particles are adjusted so that, in the presence of polynucleotides, the structure of the polynucleotides collapses and the spatial extent of the polynucleotide is reduced.

8. Nanoparticle-polynucleotide complexes according to one of Claims 1 to 7, **characterized in that** molecules which make it possible to control the biological transport pathways are coupled via the functional, derivatized or modified groups of the organosilane or polyorganosiloxane.

9. Nanoparticle-polynucleotide complexes according to one of Claims 1 to 8, **characterized in that** surface modifiers which affect the biocirculation are applied to the complex surface.

10. Nanoparticle-polynucleotide complexes according to one of Claims 1 to 9, **characterized in that** they are enclosed by a liposomal system.

11. Nanoparticle-polynucleotide complexes according to one of Claims 1 to 10 for the specific or aspecific transfer of genetic information into target cells.

12. Nanoparticle-polynucleotide complexes according to one of Claims 1 to 11 for the local, oral or systemic treatment of diseases.

13. Nanoparticle-polynucleotide complexes according to one of Claims 1 to 12 for the transfection of target cells or for gene therapy.

14. Pharmaceutical composition, containing at least one nanoparticle-polynucleotide complex according to one of Claims 1 to 13 and pharmaceutically acceptable excipients and/or auxiliaries.

15. Use of the nanoparticle-polynucleotide complexes according to one of Claims 1 to 10 for the stable storage of polynucleotides..

16. Use of the nanoparticle-polynucleotide complexes according to one of Claims 1 to 10 for the specific or aspecific transfer of genetic information into target cells or for the transfection of target cells, excluding therapeutic treatments.

## Revendications

1. Complexes nanoparticule-polynucléotide, dans lesquels des particules de l'ordre de grandeur du nanomètre, ayant un noyau constitué de particules primaires minérales dont la surface est recouverte partiellement ou totalement d'une enveloppe par réaction avec un organosilane ou un polyorganosiloxane contenant des groupes fonctionnels, grâce à quoi les particules présentent dans le domaine de pH neutre de 7,0 à 7,5, un potentiel Zêta de 0 à + 100 mV, sont liées à des polynucléotides.

2. Complexes nanoparticule-polynucléotide selon la revendication 1, **caractérisés en ce que** les particules primaires minérales sont des particules choisies parmi des oxydes métalliques comme la magnétite ou la maghémite, des oxydes non-métalliques comme SiO₂, des phosphates de calcium et des dérivés du bore.

3. Complexes nanoparticule-polynucléotide selon la revendication 1 ou 2, **caractérisés en ce que** les groupes fonctionnels de l'organosilane ou du polyorganosiloxane sont des groupes amino.

4. Complexes nanoparticule-polynucléotide selon l'une quelconque des revendications 1 à 3, **caractérisés en ce que** les organosilanes utilisés ont été transformés en des dérivés à l'aide d'agents de transformation.

5. Complexes nanoparticule-polynucléotide selon l'une quelconque des revendications 1 à 4, **caractérisés en ce que** les groupes fonctionnels ou les groupes dérivés de l'organosilane ou du polyorganosiloxane ont été mis à réagir avec des agents de modification.

6. Complexes nanoparticule-polynucléotide selon l'une quelconque des revendications 1 à 5, **caractérisés en ce que** les groupes fonctionnels, les agents de transformation et les agents de modification ont été choisis de telle manière que les particules présentent une charge superficielle positive.

7. Complexes nanoparticule-polynucléotide selon l'une quelconque des revendications 1 à 6, **caractérisés en ce que** l'on règle la densité de la charge superficielle et la taille de particule des particules de l'ordre de grandeur du nanomètre de telle manière que, en présence de polynucléotides, la structure des polynucléotides s'affaisse et l'étendue spatiale du polynucléotide diminue.

8. Complexes nanoparticule-polynucléotide selon l'une quelconque des revendications 1 à 7, **caractérisés en ce que**, par l'intermédiaire des groupes fonctionnels, dérivés ou modifiés de l'organosilane ou du polyorganosiloxane, sont couplées des molécules qui permettent un réglage des voies de transport biologiques.

9. Complexes nanoparticule-polynucléotide selon l'une quelconque des revendications 1 à 8, **caractérisés en ce que** sont appliqués sur la surface du complexe des modificateurs superficiels qui agissent sur la circulation biologique.

10. Complexes nanoparticule-polynucléotide selon l'une quelconque des revendications 1 à 9, **caractérisés en ce qu'**ils sont entourés d'un système liposomique.

11. Complexes nanoparticule-polynucléotide selon l'une quelconque des revendications 1 à 10, qui sont destinés au transfert spécifique ou non-spécifique de l'information génétique dans des cellules-cibles.

12. Complexes nanoparticule-polynucléotide selon l'une quelconque des revendications 1 à 11, qui sont destinés au traitement de maladies, par voie locale, orale ou systémique.

13. Complexes nanoparticule-polynucléotide selon l'une quelconque des revendications 1 à 12, qui sont destinés à la transfection de cellules-cibles, ou à la thérapie génétique.

14. Composition pharmaceutique qui contient au moins un complexe nanoparticule-polynucléotide selon l'une quelconque des revendications 1 à 13, et des excipients et/ou des produits auxiliaires pharmaceutiquement acceptables.

15. Utilisation des complexes nanoparticule-polynucléotide selon l'une quelconque des revendications 1 à 10, pour le stockage stable de polynucléotides.

16. Utilisation des complexes nanoparticule-polynucléotide selon l'une quelconque des revendications 1 à 10, pour le transfert spécifique ou non-spécifique de l'information génétique dans des cellules-cibles ou pour la transfection de cellules-cibles, les traitements thérapeutiques étant exclus.
